# EUROPEAN PATENT APPLICATION

(11) **EP 4 782 418 A1**
(43) Date of publication of application: **29.07.2026**
(21) Application number: 24868382.3
(22) Date of filing: 20.09.2024
(51) Int. Cl.: C04B 35/486, A61C 5/70, A61C 13/00, A61C 13/083, B01D 15/00, C01G 25/02

(54) **ZIRCONIA CALCINED BODY**

(30) Priority: 22.09.2023 JP 2023159084
(71) Applicant: Kuraray Noritake Dental Inc., Kurashiki-shi Okayama 710-0801 (JP)
(72) Inventor: KAWAI, Makito, Aichi 4700293 (JP); SAKAMOTO, Hiroyuki, Aichi 4700293 (JP); KATO, Makiko, Aichi 4700293 (JP); YAMAMOTO, Takahisa, Aichi 4700293 (JP)
(74) Representative: D Young & Co LLP
(86) International application number: PCT/JP2024/033775
(87) International publication number: WO 2025/063306

(57) **Abstract**

The present invention provides a zirconia pre-sintered body that can achieve a relative density of 99.0% or more in fast firing with a high rate of temperature increase of 350°C/min, and yield a zirconia sintered body having excellent translucency. The present invention relates to a zirconia pre-sintered body comprising zirconia and yttria, wherein the zirconia pre-sintered body, with six target temperatures set from 1,150°C to 1,400°C at 50°C intervals and six samples prepared by heating the zirconia pre-sintered body from a temperature range of 400°C or less to each different target temperature at a rate of temperature increase of 350°C/min, followed by 10-minute retention at the target temperature and subsequent cooling to 25°C, has a transition point where the maximum value of the amount of change ((Δρₙ₊₁/ΔT) - (Δρₙ/ΔT)) between the rate of density change Δρₙ/ΔT in the nth interval and the rate of density change Δρₙ₊₁/ΔT in the (n+1)th interval becomes 0.010 or more in the temperature range of 1,200°C or more and 1,350°C or less, where Δρₙ represents a change in density of the pre-sintered body between the upper-limit temperature and the lower-limit temperature of the nth interval (n being an integer of 1 or greater), and ΔT represents a change in temperature as defined for the six samples in the temperature range of target temperatures from 1,150°C to 1,400°C divided into 50°C intervals.

## Description

### TECHNICAL FIELD

The present invention relates to zirconia pre-sintered bodies. More specifically, the present invention relates to a zirconia pre-sintered body that can achieve a relative density of 99.0% or more in fast firing with a high rate of temperature increase of 350°C/min, and yield a zirconia sintered body having excellent translucency.

### BACKGROUND ART

Zirconia sintered bodies find widespread applications in industry. In particular, zirconia sintered bodies have recently been used in dental material applications such as dental prostheses. In many cases, such dental prostheses are produced from a zirconia molded body of a desired shape, such as a disc or a prism, prepared by, for example, press molding zirconia particles or molding a composition containing zirconia particles. The zirconia molded body is then pre-sintered to prepare a pre-sintered body (mill blank), which is subsequently fired after being cut (milled) into a shape of the desired dental prosthesis.

Zirconia is a compound that undergoes a phase transformation between crystal systems. In this connection, partially-stabilized zirconia (PSZ) and fully-stabilized zirconia (FSZ), which exist as solid solutions of zirconia with stabilizers such as yttria (yttrium oxide; Y₂O₃) to prevent such phase transformations, are used in a wide variety of fields.

In the field of dentistry, zirconia material has been used as a material for frameworks because zirconia material, while possessing high strength, exhibits low translucency. In recent years, however, improvements in the translucency of zirconia material have led to increased production of zirconia-only dental prostheses.

Considering that dental prostheses produced from zirconia material are excellent in strength and translucency, there have been proposed zirconia sintered bodies, for example, such as that in Patent Literature 1.

### CITATION LIST

### Patent Literature

Patent Literature 1: JP 2010-150064 A

### SUMMARY OF INVENTION

### Technical Problem

Patent Literature 1 discloses that keeping the sintering shrinkage rate (Δρ/ΔT: g/cm³·°C) between 0.0120 and 0.0135 in the relative density range of 70% to 90% during firing at 5°C/min can improve the density of the resulting zirconia sintered body.

However, Patent Literature 1 does not address the density change of the sintered body under rates of temperature increase faster than 5°C/min.

Concerning the invention according to Patent Literature 1, the present inventors have also found that the sintered body, when heated at an increased rate (350°C/min) and retained for a certain time period, does not always achieve a relative density of 99.0% or more, failing to exhibit the translucency achieved by firing at 5°C/min. That is, it was revealed that there remain areas for improvement with respect to further reduction of firing time in the invention according to Patent Literature 1.

It is an object of the present invention to provide a zirconia pre-sintered body that can achieve a relative density of 99.0% or more in fast firing with a high rate of temperature increase of 350°C/min, and yield a zirconia sintered body having excellent translucency.

### Solution to Problem

The present inventors conducted intensive research to find a solution to the foregoing issues. In the investigation, six target temperatures were set from 1,150°C to 1,400°C at 50°C intervals, and six samples were prepared by heating a zirconia pre-sintered body comprising zirconia and yttria from a temperature range of 400°C or less to each different target temperature at a rate of temperature increase of 350°C/min, followed by 10-minute retention at the target temperature and subsequent cooling to 25°C. The temperature range of target temperatures from 1,150°C to 1,400°C was divided into 50°C intervals. Here, the temperature intervals were set in steps of 50°C, beginning at 1,150°C, with the temperature range from 1,150°C to 1,200°C defining the first interval, the temperature range from 1,200°C to 1,250°C defining the second interval, and each subsequent range defined as the nth interval (n being an integer of 1 or greater). It was found that a zirconia sintered body that exhibits excellent translucency even after fast firing with a high rate of temperature increase of 350°C/min can be obtained when the zirconia pre-sintered body has a transition point where the maximum value of the amount of change ((Δρₙ₊₁/ΔT) - (Δρₙ/ΔT)) between the rate of density change Δρₙ/ΔT in the nth interval and the rate of density change Δρₙ₊₁/ΔT in the (n+1)th interval becomes 0.010 or more in the temperature range of 1,200°C or more and 1,350°C or less, where Δρₙ represents a change in density of the pre-sintered body between the upper-limit temperature and the lower-limit temperature of the nth interval (n being an integer of 1 or greater), and ΔT represents a change in temperature as defined for the six samples. Building upon this finding, the present inventors conducted additional examinations, ultimately resulting in the completion of the present invention.

The present invention includes the following.
[1] A zirconia pre-sintered body comprising zirconia and yttria,
   wherein the zirconia pre-sintered body, with six target temperatures set from 1,150°C to 1,400°C at 50°C intervals and six samples prepared by heating the zirconia pre-sintered body from a temperature range of 400°C or less to each different target temperature at a rate of temperature increase of 350°C/min, followed by 10-minute retention at the target temperature and subsequent cooling to 25°C, has a transition point where the maximum value of the amount of change ((Δρₙ₊₁/ΔT) - (Δρₙ/ΔT)) between the rate of density change Δρₙ/ΔT in the nth interval and the rate of density change Δρₙ₊₁/ΔT in the (n+1)th interval becomes 0.010 or more in the temperature range of 1,200°C or more and 1,350°C or less, where Δρₙ represents a change in density of the pre-sintered body between the upper-limit temperature and the lower-limit temperature of the nth interval (n being an integer of 1 or greater), and ΔT represents a change in temperature as defined for the six samples in the temperature range of target temperatures from 1,150°C to 1,400°C divided into 50°C intervals.
[2] The zirconia pre-sintered body according to [1], wherein the yttria content is 3.5 mol% or more and 7.5 mol% or less relative to the total moles of the zirconia and the yttria.
[3] The zirconia pre-sintered body according to [1] or [2], which has a density of 4.5 g/cm³ or less at the temperature (°C) of the transition point.

### Advantageous Effects of Invention

According to a zirconia pre-sintered body of the present invention, a zirconia pre-sintered body can be provided that can achieve a relative density of 99.0% or more in fast firing with a high rate of temperature increase of 350°C/min, and yield a zirconia sintered body having excellent translucency.

The present invention can also provide a zirconia pre-sintered body that can yield a zirconia sintered body with excellent translucency even with a hold time of 2 minutes at the highest sintering temperature.

### DESCRIPTION OF EMBODIMENTS

### [Zirconia Pre-Sintered Body]

A zirconia pre-sintered body of the present invention comprises zirconia and yttria, wherein the zirconia pre-sintered body, with six target temperatures set from 1,150°C to 1,400°C at 50°C intervals and six samples prepared by heating the zirconia pre-sintered body from a temperature range of 400°C or less to each different target temperature at a rate of temperature increase of 350°C/min, followed by 10-minute retention at the target temperature and subsequent cooling to 25°C, has a transition point where the maximum value of the amount of change ((Δρₙ₊₁/ΔT) - (Δρₙ/ΔT)) between the rate of density change Δρₙ/ΔT in the nth interval and the rate of density change Δρₙ₊₁/ΔT in the (n+1)th interval becomes 0.010 or more in the temperature range of 1,200°C or more and 1,350°C or less, where Δρₙ represents a change in density of the pre-sintered body between the upper-limit temperature and the lower-limit temperature of the nth interval (n being an integer of 1 or greater), and ΔT represents a change in temperature as defined for the six samples in the temperature range of target temperatures from 1,150°C to 1,400°C divided into 50°C intervals.

As used herein, a "zirconia composition" refers to a composition comprising a zirconia powder and a stabilizer powder.

As used herein, a "molded body" refers to an object that has not reached a semi-sintered state (pre-sintered state) or a sintered state. That is, "molded body" distinguishes itself from pre-sintered body and sintered body in that it remains unfired after being molded.

As used herein, a "zirconia pre-sintered body" refers to an object in a semi-sintered state where zirconia particles have formed necks between particles but have not fully sintered.

As used herein, a "zirconia sintered body" refers to an object in a sintered state where zirconia particles have fully sintered. In a zirconia sintered body, sintering has caused zirconia particles to consolidate, increasing the relative density and promoting densification, resulting in a fully sintered state with a relative density of 95% or more.

As used herein, "zirconia" refers to zirconium(IV) oxide (ZrO₂), with ZrO₂ particles containing a trace amount (0.5 mass% or more and 3 mass% or less) of HfO₂ relative to the amount of ZrO₂. Because HfO₂ is difficult to separate, terms such as "zirconia", "zirconia particles", and "zirconia powder" are intended to include both ZrO₂ and HfO₂. Likewise, particles or powders with the stabilizer present in zirconia as a solid solution are also encompassed by "zirconia particles" or "zirconia powders."

As used herein, the term "ordinary pressure" means standard atmospheric pressure (1 atm).

As used herein, "theoretical density" refers to the density determined from the volume of a crystal unit cell and the total mass contained in the unit cell. For example, the theoretical density is 6.095 g/cm³ for 3 mol% yttria-stabilized zirconia, and 6.052 g/cm³ for 5.5 mol% yttria-stabilized zirconia.

As used herein, the term "transition point" refers to a point at which the difference (amount of change) in the rate of density change between adjacent 50°C intervals within a specific temperature range (described separately) takes a value other than zero. The temperature of the transition point between the nth interval and (n+1)th interval refers to the upper-limit temperature of the nth interval at which the value of (Δρₙ₊₁/ΔT) - (Δρₙ/ΔT) becomes nonzero.

In this specification, the upper limits and lower limits of numeric ranges (for example, such as temperature ranges, the content of each component, the abundance of crystal systems, values calculated from components, and various physical properties) can be appropriately combined.

In view of achieving a relative density of 99.0% or more in fast firing with a high rate of temperature increase of 350°C/min and providing a zirconia sintered body that exhibits greater translucency, the amount of change ((Δρₙ₊₁/ΔT) - (Δρₙ/ΔT)) between the rate of density change Δρₙ/ΔT in the nth interval and the rate of density change Δρₙ₊₁/ΔT in the (n+1)th interval in the temperature range of 1,200°C or more and 1,350°C or less is preferably 0.010 or more, more preferably 0.011 or more, even more preferably 0.012 or more.

In view of providing a zirconia sintered body that exhibits greater translucency in fast firing with a high rate of temperature increase of 350°C/min, the amount of change ((Δρₙ₊₁/ΔT) - (Δρₙ/ΔT)) is preferably 0.050 or less, more preferably 0.045 or less, even more preferably 0.040 or less.

The measurement method for the density of the pre-sintered body and the method for calculating the density change are as described in the EXAMPLES below.

With the amount of change ((Δρₙ₊₁/ΔT) - (Δρₙ/ΔT)) falling in the predetermined ranges in a zirconia pre-sintered body of the present invention, it is possible to achieve a relative density of 99.0% or more in fast firing with a high rate of temperature increase of 350°C/min, and provide a zirconia sintered body that exhibits excellent translucency. While the exact reason remains unclear, the following speculation has been made.

Apparently, fast firing with a high rate of temperature increase of 350°C/min (hereinafter, also referred to simply as "fast firing") yields a sintered body of low relative density containing internal pores.

The present inventors have speculated that providing a transition point where the amount of change ((Δρₙ₊₁/ΔT) - (Δρₙ/ΔT)) takes a maximum value of 0.010 or more in the temperature range of 1,200°C or more and 1,350°C or less in the aforementioned fast firing might inhibit rapid grain growth in the temperature range preceding the transition point, reducing pore entrapment in the low temperature range of 1,200°C or more and 1,350°C or less. It was considered that, in the higher temperature range above 1,350°C, the density difference occurring between phases as the zirconia raw material undergoes crystal phase transformations (for example, from monoclinic to tetragonal or cubic crystal) facilitates the removal of voids.

This probably explains why the resulting sintered body can achieve a relative density of 99.0% or more and exhibit excellent translucency despite fast firing.

In a zirconia pre-sintered body of the present invention, in view of further facilitating the removal of voids in the high temperature range, the density of the pre-sintered body at the temperature (°C) of the transition point (where the amount of change takes the maximum value) is preferably 4.5 g/cm³ or less, more preferably 4.2 g/cm³ or less, even more preferably 4.1 g/cm³ or less.

In view of achieving a relative density of 99.0% or more in fast firing, the density of the pre-sintered body at the temperature (°C) of the transition point is preferably 3.0 g/cm³ or more, more preferably 3.1 g/cm³ or more, even more preferably 3.2 g/cm³ or more.

The zirconia pre-sintered body comprises yttria (Y₂O₃) as a stabilizer capable of preventing a phase transformation of zirconia.

The yttria content in the zirconia pre-sintered body is preferably 2.5 mol% or more, more preferably 3.0 mol% or more, even more preferably 3.5 mol% or more, particularly preferably 4.0 mol% or more relative to the total moles of zirconia (zirconium(IV) oxide, or ZrO₂) and the stabilizer. A yttria content of 2.5 mol% or more is preferred in terms of increasing the cubic system within the crystal systems of the sintered body and improving translucency.

The stabilizer content is preferably 10 mol% or less, more preferably 9.0 mol% or less, even more preferably 8.5 mol% or less, particularly preferably 8 mol% or less. A stabilizer content of 10 mol% or less is preferred in terms of preventing a decrease in strength.

In a zirconia pre-sintered body of the present invention, the stabilizer capable of preventing a phase transformation of zirconia may be solely yttria. Alternatively, a zirconia pre-sintered body of the present invention may additionally comprise another stabilizer, other than yttria, capable of preventing a phase transformation of zirconia.

Examples of such non-yttria stabilizers capable of preventing a phase transformation of zirconia (hereinafter, also referred to simply as "stabilizers") include oxides such as calcium oxide (CaO), magnesium oxide (MgO), yttrium oxide (Y₂O₃), cerium oxide (CeO₂), scandium oxide (Sc₂O₃), niobium oxide (Nb₂O₅), lanthanum oxide (La₂O₃), erbium oxide (Er₂O₃), praseodymium oxide (Pr₂O₃, Pr₆O₁₁), samarium oxide (Sm₂O₃), europium oxide (Eu₂O₃), thulium oxide (Tm₂O₃), gallium oxide (Ga₂O₃), indium oxide (In₂O₃), and ytterbium oxide (Yb₂O₃). The stabilizer may be used alone, or two or more thereof may be used in combination.

A zirconia pre-sintered body of the present invention may comprise additives other than zirconia and stabilizers, provided that the present invention can exhibit its effects. Examples of such additives include colorants (including pigments, composite pigments, and fluorescent agents), alumina (Al₂O₃), titanium oxide (TiO₂), and silica (SiO₂).

Examples of the pigments include oxides of at least one element selected from the group consisting of Ti, V, Cr, Mn, Fe, Co, Ni, Zn, Y, Sn, Sb, Bi, Ce, Pr, Sm, Eu, Gd, Tb, and Er (specifically, such as NiO and Cr₂O₃), excluding Y₂O₃ and CeO₂, preferably oxides of at least one element selected from the group consisting of Ti, V, Cr, Mn, Fe, Co, Ni, Zn, Y, Sn, Sb, Bi, Ce, Pr, Sm, Eu, Gd, and Tb, more preferably oxides of at least one element selected from the group consisting of Ti, V, Cr, Mn, Fe, Co, Ni, Zn, Y, Sn, Sb, Bi, Ce, Sm, Eu, Gd, and Tb.

A zirconia pre-sintered body of the present invention may be one that does not comprise erbium oxide (Er₂O₃).

Examples of the composite pigments include (Zr,V)O₂, Fe(Fe,Cr)₂O₄, (Ni,Co,Fe)(Fe,Cr)₂O₄·ZrSiO₄, and (Co,Zn)Al₂O₄. Examples of the fluorescent agents include Y₂SiO₅:Ce, Y₂SiO₅:Tb, (Y,Gd,Eu)BO₃, Y₂O₃:Eu, YAG:Ce, ZnGa₂O₄:Zn, and BaMgAl₁₀O₁₇:Eu.

A zirconia pre-sintered body of the present invention may comprise a fluorescent agent. The zirconia sintered body can exhibit fluorescence by incorporating a fluorescent agent in the zirconia pre-sintered body. The type of fluorescent agent is not particularly limited, and it is possible to use a single fluorescent agent or two or more fluorescent agents capable of emitting fluorescence at any wavelength of light.

Examples of the fluorescent agent include those containing metal elements. Examples of the metal elements include Ga, Bi, Ce, Nd, Sm, Eu, Gd, Tb, Dy, and Tm. The fluorescent agent may contain one of these metal elements by itself, or may contain two or more of these metal elements. Preferred among these metal elements are Ga, Bi, Eu, Gd, and Tm, more preferably Bi and Eu.

Examples of such fluorescent agents include oxides, hydroxides, acetates, and nitrates of the metal elements above. The fluorescent agent may be, for example, Y₂SiO₅:Ce, Y₂SiO₅:Tb, (Y,Gd,Eu)BO₃, Y₂O₃:Eu, YAG:Ce, ZnGa₂O₄:Zn, or BaMgAl₁₀O₁₇:Eu.

The content of the fluorescent agent in the zirconia pre-sintered body is not particularly limited, and may be appropriately adjusted according to factors such as the type of fluorescent agent, and the intended use of the zirconia sintered body. However, in view of considerations such as suitability as dental prostheses, the content of fluorescent agent is preferably 0.001 mass% or more, more preferably 0.005 mass% or more, even more preferably 0.01 mass% or more in terms of the oxide equivalent of the metal elements contained in the fluorescent agent, relative to 100 mass% of the zirconia contained in the zirconia pre-sintered body.

The content of fluorescent agent is not particularly limited, and may be 1 mass% or less, 0.5 mass% or less, or 0.1 mass% or less in terms of the oxide equivalent of the metal elements contained in the fluorescent agent, provided that the fluorescent agent can exhibit suitable fluorescence. When the content is at or above these lower limits, the fluorescence can match or exceed that of natural human teeth. When the content is at or below the foregoing upper limits, it is possible to reduce a decrease in translucency and mechanical strength.

In order to ensure strength that enables mechanical processing in a pre-sintered state, the flexural strength of a zirconia pre-sintered body of the present invention is preferably 15 MPa or more. For ease of mechanical processing in a pre-sintered state, the flexural strength of the zirconia pre-sintered body is preferably 70 MPa or less, more preferably 60 MPa or less.

The flexural strength can be measured in compliance with ISO 6872:2015 (Dentistry-Ceramic materials). For measurement, specimens measuring 5 mm × 10 mm × 50 mm are used under the same conditions except for size. For surface finishing, the specimen surfaces, including the chamfered surface (45° chamfers at the corners of specimen), are finished longitudinally with #600 sandpaper. The specimen is disposed in such an orientation that its widest face is perpendicular to the vertical direction (loading direction). In the three-point flexural test, measurement is carried out at a crosshead speed of 0.5 mm/min with the distance between supports (span) set at 30 mm.

A zirconia pre-sintered body of the present invention has a density of preferably 2.7 g/cm³ or more, more preferably 3.0 g/cm³ or more, even more preferably 3.2 g/cm³ or more.

The zirconia pre-sintered body has a density of preferably 4.0 g/cm³ or less, more preferably 3.8 g/cm³ or less, even more preferably 3.6 g/cm³ or less. A density in these ranges allows for easy molding.

The density of the zirconia pre-sintered body can be calculated as the mass-to-volume ratio (mass of zirconia pre-sintered body) / (volume of zirconia pre-sintered body). For example, the density of the zirconia pre-sintered body can be determined by cutting out test specimens with dimensions of 10 mm × 10 mm × 10 mm from arbitrary locations of the zirconia pre-sintered body (n = 3), measuring the mass and volume of each test specimen, and averaging the measured values. The calculated arithmetic average can then be applied to the above formula to determine the density.

### [Production Method of Zirconia Pre-Sintered Body]

The following describes a method for producing the zirconia pre-sintered body.

The production method of the zirconia pre-sintered body is not particularly limited, as long as it can provide the desired transition point. In a preferred production method of the zirconia pre-sintered body, a zirconia pre-sintered body of the present invention can be produced by pulverizing a zirconia composition, and firing (pre-sintering) a molded body of the pulverized zirconia composition to an extent that the zirconia particles do not sinter, with the pulverization process providing excess energy under predetermined conditions compared to conventional techniques.

The zirconia composition can be produced by mixing the raw material zirconia powder and yttria powder. The mixing method is not particularly limited, and any known method and apparatus may be used.

The zirconia and yttria particles constituting their respective powders may be prepared using a method, for example, such as the breakdown process, in which coarse particles are pulverized or crushed into a fine powder, or the building-up process, which synthesizes particles through nucleation and growth from atoms or ions.

The zirconia constituting the zirconia powder is not particularly limited, and may be any of tetragonal zirconia, monoclinic zirconia, or cubic zirconia.

These may be present alone, or two or more thereof may be present in combination in the zirconia powder.

The zirconia powder may employ commercially available zirconia particles, or a commercially available powder may be used after pulverization with a known pulverizing mixer (such as a ball mill).

The yttria powder may employ commercially available yttria particles, or a commercially available powder may be used after pulverization with a known pulverizing mixer (such as a ball mill).

Examples of commercially available zirconia powders include zirconia powder products such as those manufactured by Tosoh Corporation under various trade names, including Zpex^{®} (Y₂O₃ content: 3 mol%), Zpex^{®} 4 (Y₂O₃ content: 4 mol%), Zpex^{®} Smile^{®} (Y₂O₃ content: 5.5 mol%), TZ-3Y (Y₂O₃ content: 3 mol%), TZ-3YS (Y₂O₃ content: 3 mol%), TZ-4YS (Y₂O₃ content: 4 mol%), TZ-6Y (Y₂O₃ content: 6 mol%), TZ-6YS (Y₂O₃ content: 6 mol%), TZ-8YS (Y₂O₃ content: 8 mol%), TZ-10YS (Y₂O₃ content: 10 mol%), TZ-3Y-E (Y₂O₃ content: 3 mol%), TZ-3YS-E (Y₂O₃ content: 3 mol%), TZ-3YB-E (Y₂O₃ content: 3 mol%), TZ-3YSB-E (Y₂O₃ content: 3 mol%), TZ-3YB (Y₂O₃ content: 3 mol%), TZ-3YSB (Y₂O₃ content: 3 mol%), TZ-3Y20AB (Y₂O₃ content: 3 mol%), TZ-8YSB (Y₂O₃ content: 8 mol%), and TZ-0 (Y₂O₃ content: 0 mol%; monoclinic zirconia).

The zirconia composition is pulverized further. The desired transition point can be provided by subjecting the zirconia composition to a pulverization process under predetermined conditions.

In the present invention, subjecting the zirconia composition containing zirconia and yttria to a pulverization process under predetermined conditions facilitates mixing of zirconia particles and yttria particles, resulting in a uniform compositional distribution. This limits the temperature range in which zirconia undergoes phase transformations, providing the desired transition point not observed in conventionally available products. The presence of the transition point inhibits rapid grain growth in the temperature range preceding the transition point, reducing pore entrapment in the low temperature range of 1,200°C or more and 1,350°C or less. In the higher temperature range above 1,350°C, the density difference occurring between phases as the zirconia raw material undergoes crystal phase transformations (for example, from monoclinic to tetragonal or cubic crystal) facilitates the removal of voids. Presumably, this leads to excellent translucency even with a hold time of 2 minutes at the highest sintering temperature.

Preferably, the pulverization process employs the following method.
(i) pulverization for a period of 10 minutes or more using a pulverization medium of less than 1 mm in diameter, or (ii) pulverization for a period of more than 40 hours using a pulverization medium of 1 mm or more in diameter.

Pulverization media with fine diameters help provide the desired transition point. From this viewpoint, when using a pulverization medium of less than 1 mm in diameter, the size (diameter) of the pulverization medium is preferably 0.1 to 0.5 mm.

The pulverization medium of less than 1 mm in diameter may be a commercially available product.

The pulverization medium of less than 1 mm in diameter may be used with a pulverization device, for example, such as a bead mill.

When using a pulverization medium of less than 1 mm in diameter, the duration of the pulverization process is not particularly limited. However, in view of allowing easier pulverization of yttria particles into fine particle sizes, the pulverization process is preferably 10 minutes or more, more preferably 12 minutes or more, even more preferably 15 minutes or more, particularly preferably 20 minutes or more. The pulverization process is preferably 20 hours or less, more preferably 10 hours or less, even more preferably 5 hours or less, particularly preferably 2 hours or less.

When the pulverization device is a device that operates by circulating slurry (for example, a bead mill), the duration of the pulverization process refers to the period for which the slurry remains in the vessel (pulverization chamber).

In view of providing a long pulverization time and more readily increasing the energy level in the pulverization process in combination with the extended pulverization time, the size (diameter) of pulverization medium is preferably 1.5 mm or more, more preferably 2.0 mm or more when using a pulverization medium of 1 mm or more in diameter.

The pulverization medium of 1 mm or more in diameter may be a commercially available product.

The pulverization medium of 1 mm or more in diameter may be used with a pulverization device, for example, such as a ball mill.

When using a pulverization medium of 1 mm or more in diameter, the pulverization time (the duration of the pulverization process) is not particularly limited. However, the pulverization time is preferably more than 40 hours, more preferably 55 hours or more. In view of more readily providing the desired transition point by facilitating mixing of zirconia particles and yttria particles and providing a uniform compositional distribution in pulverizing the zirconia composition under applied energy in excess of that conventionally employed, the pulverization time is even more preferably 80 hours or more, particularly preferably 100 hours or more. The duration of the pulverization process is preferably 1,000 hours or less, more preferably 800 hours or less, even more preferably 500 hours or less, particularly preferably 300 hours or less.

The zirconia composition may have various forms, including granules and slurry.

When the zirconia composition is a slurry, it can be produced by mixing the mixed powder from the pulverization process with a solvent (preferably water).

In view of providing a sintered body having excellent mechanical strength and translucency, the average particle diameter of the zirconia composition after pulverization is preferably 0.2 µm or less.

The average particle diameter of zirconia particles and yttria particles can be measured using a dynamic light scattering particle size distribution measurement method. For example, measurement may be conducted using a dynamic light scattering particle size distribution analyzer (manufactured by Horiba Ltd. under the trade name SZ-100V2), where a slurry diluted with water to about 0.1 mass% is subjected to ultrasonic waves for 30 minutes, and subsequently measured by volume under continued ultrasonic radiation.

The zirconia composition may additionally comprise additives such as binders, dispersants, emulsifiers, antifoaming agents, pH adjusters, lubricants, and translucency adjusters. The additives may be used alone, or two or more thereof may be used in combination.

Examples of the binders include polyvinyl alcohol, methyl cellulose, carboxymethyl cellulose, acrylic binders, wax binders, polyvinyl butyral, polymethyl methacrylate, and ethyl cellulose.

For improved translucency, the binder content in a zirconia composition of the present invention is preferably 10 mass% or less, more preferably 7 mass% or less, even more preferably 5 mass% or less relative to 100 mass% of zirconia.

Examples of the plasticizers include polyethylene glycol, glycerin, propylene glycol, and dibutyl phthalate.

Examples of the dispersants include ammonium polycarboxylate (such as triammonium citrate), ammonium polyacrylate, acryl copolymer resins, acrylic acid ester copolymers, polyacrylic acid, bentonite, carboxymethyl cellulose, anionic surfactants (for example, polyoxyethylene alkyl ether phosphoric acid esters such as polyoxyethylene lauryl ether phosphoric acid ester), non-ionic surfactants, oleic glyceride, amine salt surfactants, oligosaccharide alcohols, and stearic acid.

Examples of the emulsifiers include alkyl ethers, phenyl ethers, and sorbitan derivatives.

Examples of the antifoaming agents include alcohols, polyethers, polyethylene glycols, silicone, and waxes.

Examples of the pH adjusters include ammonia, ammonium salts (including ammonium hydroxides such as tetramethylammonium hydroxide).

Examples of the lubricants include polyoxyethylene alkyl ethers, and waxes.

Examples of the translucency adjusters include aluminum oxide (Al₂O₃), titanium oxide (TiO₂), silicon dioxide (SiO₂), zircon, lithium silicate, and lithium disilicate.

The particles constituting the zirconia composition have a BET specific surface area of preferably 7.0 m²/g or more, more preferably 7.5 m²/g or more, even more preferably 8.0 m²/g or more as measured in compliance with JIS Z 8830:2013. When the BET specific surface area is 7.0 m²/g or more, it is possible to inhibit opacification of the sintered body upon sintering. The BET specific surface area is preferably 50 m²/g or less, more preferably 45 m²/g or less, even more preferably 40 m²/g or less. A BET specific surface area of 50 m²/g or less reduces susceptibility to the influence of temperature variations inside the furnace.

The BET specific surface area can be measured using a commercially available product, such as a full automatic specific surface area analyzer (manufactured by Mountech Co., Ltd. under the trade name Macsorb^{®} HM model-1200; BET flow method (single point / multi point)). For example, measurement may be conducted using this full automatic specific surface area analyzer following the BET flow method (single point).

The translucency of the sintered body also becomes less likely to decrease with a shorter firing period in sintering. Here, a "BET specific surface area" is a specific surface area measured without distinguishing primary particles and secondary particles.

The next step is the molding process, where the pulverized zirconia composition is molded to prepare a molded body. The molded body can be obtained by molding the zirconia composition under applied external force using a known method.

The molding method is not particularly limited, and may employ the following methods, for example.
(a) slip casting of a slurry containing the pulverized zirconia composition
(b) gel casting of a slurry containing the pulverized zirconia composition
(c) press molding of the pulverized zirconia composition
(d) molding of a zirconia composition containing zirconia particles, yttria particles, and resin
(e) polymerization of a zirconia composition containing zirconia particles, yttria particles, and a polymerizable monomer or oligomer
(f) additive fabrication of granules containing zirconia particles and yttria particles

### (a) Slip Casting

When the zirconia molded body is produced using a method that includes the step of slip casting a slurry containing the pulverized zirconia composition, the slip casting is not particularly limited to specific methods, and may be carried out using, for example, a method that dries the slurry after pouring it in a mold.

For considerations such as ease of pouring the slurry into a mold, and increasing the reusability of the mold in addition to preventing excessive drying time, the content of the dispersion medium in the slurry used is preferably 80 mass% or less, more preferably 50 mass% or less, even more preferably 20 mass% or less.

The slurry may be poured into a mold under ordinary pressure. However, in view of production efficiency, it is preferable to pour the slurry under applied pressure conditions.

### (b) Gel Casting

When the zirconia molded body is produced using a method that includes the step of gel casting a slurry containing the pulverized zirconia composition, the gel casting is not particularly limited to specific methods, and may be carried out using, for example, a method that dries a moist body shaped in a mold by gelling the slurry.

For considerations such as preventing excessive drying time and reducing cracking during drying, the content of the dispersion medium contained in the slurry used is preferably 80 mass% or less, more preferably 50 mass% or less, even more preferably 20 mass% or less.

The gelation may be achieved by, for example, adding a gelatinizer, or by adding and polymerizing a polymerizable monomer.

The gelatinizer is not particularly limited, and may be, for example, a water-soluble gelatinizer. Specifically, preferred are agarose and gelatin, for example. The gelatinizer may be used alone, or two or more thereof may be used in combination. The gelatinizer may be used in any amount, as long as it does not cause problems such as cracking during sintering. The gelatinizer may be used in an amount of 10 mass% or less, 5 mass% or less, or 1 mass% or less, based on the mass the slurry after the addition of gelatinizer.

The polymerizable monomer is not particularly limited, and may be, for example, a (meth)acrylate monomer such as 2-hydroxyethyl (meth)acrylate, 3-hydroxypropyl (meth)acrylate, 4-hydroxybutyl (meth)acrylate, 6-hydroxyhexyl (meth)acrylate, 10-hydroxydecyl (meth)acrylate, propylene glycol mono(meth)acrylate, glycerol mono(meth)acrylate, or erythritol mono(meth)acrylate; or a (meth)acrylamide monomer such as N-methylol(meth)acrylamide, N-hydroxyethyl(meth)acrylamide, or N,N-bis(2-hydroxyethyl)(meth)acrylamide. The polymerizable monomer may be used alone, or two or more thereof may be used in combination.

The polymerizable monomer may be used in any amount, as long as it does not cause problems such as cracking during sintering. The polymerizable monomer may be used in an amount of 10 mass% or less, 5 mass% or less, or 1 mass% or less, based on the mass the slurry after the addition of polymerizable monomer.

When gelation is performed through polymerization of a polymerizable monomer, it is preferable to use a polymerization initiator for polymerization. The polymerization initiator is not particularly limited. Particularly preferred are photopolymerization initiators. The photopolymerization initiator can be appropriately selected from photopolymerization initiators used in industry, preferably those used in dentistry.

Specific examples of photopolymerization initiators include (bis)acylphosphine oxides (including salts), thioxanthones (including salts such as quaternary ammonium salts), ketals, α-diketones, coumarins, anthraquinones, benzoin alkyl ether compounds, and α-aminoketone compounds. The photopolymerization initiators may be used alone, or two or more thereof may be used in combination. Preferred among these photopolymerization initiators is at least one selected from the group consisting of (bis)acylphosphine oxides and α-diketones. With these photopolymerization initiators, it is possible to achieve polymerization (gelation) both in the ultraviolet region (including the near ultraviolet region) and the visible region. Specifically, polymerization (gelation) can sufficiently take place irrespective of whether the light source used is a laser such as an Ar laser or a He-Cd laser, or a lamp such as a halogen lamp, a xenon lamp, a metal halide lamp, a light emitting diode (LED), a mercury lamp, or a fluorescent lamp.

The method used to dry the shaped moist body is not particularly limited, and may be, for example, natural drying, hot-air drying, vacuum drying, drying by dielectric heating, drying by induction heating, or drying under constant temperature and humidity. These methods may be used alone, or two or more thereof may be used in combination. In view of considerations such as the capability to reduce cracking during drying, preferred are natural drying, drying by dielectric heating, drying by induction heating, and drying under constant temperature and humidity.

The molds used for the slip casting and gel casting are not particularly limited, and may be, for example, porous molds made of a material such as plaster, resin, or ceramic, or non-porous molds made of a material such as metal or resin.

### (c) Press Molding

In the press molding of the pulverized zirconia composition, the press molding is not particularly limited to specific methods, and may be carried out using a known press molding machine.

Specific examples of the press molding method include uniaxial pressing.

The press molding may be conducted in multiple stages. For example, the zirconia composition after press molding may be subjected to CIP (Cold Isostatic Pressing).

The shape of the molded body is not particularly limited, and may be disk-shaped, cuboidal, or the form of a dental product (for example, a crown shape).

The molded body may be, for example, a columnar zirconia molded body formed by filling a mold with the zirconia composition (for example, granules) and compacting it by uniaxial pressing.

The density of the molded body increases with higher surface pressure in press molding. However, the zirconia molded body yields a hard zirconia pre-sintered body when its density is too high. Accordingly, the surface pressure of press molding for the preparation of the zirconia molded body is preferably 30 to 200 MPa. When the surface pressure is 30 MPa or more, superior shape retention can be achieved in the zirconia molded body, whereas a surface pressure of 200 MPa or less prevents the density of the zirconia molded body from becoming too high and helps avoid excessive hardening more effectively.

The molded body includes those densified through high-temperature pressing such as CIP (Cold Isostatic Pressing).

From the same perspectives described above, the CIP pressure is preferably 30 to 200 MPa.

### (d) Molding of Zirconia Composition Containing Resin

When the zirconia molded body is produced using a method that comprises the step of molding a zirconia composition containing zirconia particles, yttria particles, and resin, the method used to mold the zirconia composition is not particularly limited to specific methods, and may be, for example, injection molding, cast molding, or extrusion molding.

It is also possible to use additive fabrication techniques (such as 3D printing) to form the composition, including, for example, fused deposition modeling (FDM), the inkjet method, and the powder/binder lamination method. Preferred among these molding methods are injection molding and cast molding, more preferably injection molding.

The resin is not particularly limited, and is preferably the binder described above.

### (e) Polymerization of Zirconia Composition Containing Zirconia Particles, Yttria Particles, and Polymerizable Monomer or Oligomer

By polymerizing a zirconia composition containing zirconia particles, yttria particles, and a polymerizable monomer or oligomer, the polymerizable monomer or oligomer in the zirconia composition can polymerize, allowing the composition to cure.

When the zirconia molded body is produced using a method that comprises such a polymerization step, the method is not particularly limited to specific methods, and may be, for example, a method by which a zirconia composition is polymerized in a mold, or stereolithography (SLA) using a zirconia composition. The preferred method is stereolithography (SLA).

Stereolithography enables the zirconia molded body to have a shape corresponding to the shape desired for the final zirconia sintered body, at the time of its production. This makes stereolithography a potentially preferred method in certain situations, particularly when the zirconia sintered body is used as a dental material such as a dental prosthesis.

The polymerizable monomer is not particularly limited, and may be a monofunctional polymerizable monomer such as a monofunctional (meth)acrylate or a monofunctional (meth)acrylamide, or a polyfunctional polymerizable monomer such as a bifunctional aromatic compound, a bifunctional aliphatic compound, or a tri- or higher-functional compound. The polymerizable monomer may be used alone, or two or more thereof may be used in combination. Preferred for use are polyfunctional polymerizable monomers, particularly in situations where stereolithography is employed.

The oligomer is not particularly limited, as long as it is a polymerizable compound formed by the bonding of two or more of the aforementioned polymerizable monomers.

Examples of the monofunctional (meth)acrylate include:
(meth)acrylates having a hydroxyl group(s), such as 2-hydroxyethyl (meth)acrylate, 2-hydroxypropyl (meth)acrylate, 3-hydroxypropyl (meth)acrylate, 4-hydroxybutyl (meth)acrylate, 6-hydroxyhexyl (meth)acrylate, 10-hydroxydecyl (meth)acrylate, propylene glycol mono(meth)acrylate, glycerol mono(meth)acrylate, and erythritol mono(meth)acrylate;
alkyl (meth)acrylates such as methyl (meth)acrylate, ethyl (meth)acrylate, propyl (meth)acrylate, isopropyl (meth)acrylate, n-butyl (meth)acrylate, sec-butyl (meth)acrylate, t-butyl (meth)acrylate, isobutyl (meth)acrylate, n-hexyl (meth)acrylate, lauryl (meth)acrylate, cetyl (meth)acrylate, and stearyl (meth)acrylate;
alicyclic (meth)acrylates such as cyclohexyl (meth)acrylate, and isobornyl (meth)acrylate;
aromatic group-containing (meth)acrylates such as benzyl (meth)acrylate, and phenyl (meth)acrylate; and
(meth)acrylates having functional groups, such as 2,3-dibromopropyl (meth)acrylate, 3-(meth)acryloyloxypropyltrimethoxysilane, and 11-(meth)acryloyloxyundecyltrimethoxysilane.

Examples of the monofunctional (meth)acrylamide include (meth)acrylamide, N-(meth)acryloylmorpholine, N,N-dimethyl(meth)acrylamide, N,N-diethyl(meth)acrylamide, N,N-di-n-propyl(meth)acrylamide, N,N-di-n-butyl(meth)acrylamide, N,N-di-n-hexyl(meth)acrylamide, N,N-di-n-octyl(meth)acrylamide, N,N-di-2-ethylhexyl(meth)acrylamide, N-hydroxyethyl(meth)acrylamide, and N,N-bis(2-hydroxyethyl)(meth)acrylamide.

In view of excellent polymerizability, preferred among these monofunctional polymerizable monomers are (meth)acrylamides, more preferably N-(meth)acryloylmorpholine, N,N-dimethyl(meth)acrylamide, and N,N-diethyl(meth)acrylamide.

Examples of the bifunctional aromatic compound include (meth)acrylates such as 2,2-bis((meth)acryloyloxyphenyl)propane, 2,2-bis[4-(2-hydroxy-3-acryloyloxy-2-hydroxypropoxy)phenyl]propane, 2,2-bis[4-(2-hydroxy-3-methacryloyloxypropoxy)phenyl]propane (commonly known as Bis-GMA), 2,2-bis(4-(meth)acryloyloxyethoxyphenyl)propane, 2,2-bis(4-(meth)acryloyloxypolyethoxyphenyl)propane, 2,2-bis(4-(meth)acryloyloxydiethoxyphenyl)propane, 2,2-bis(4-(meth)acryloyloxytetraethoxyphenyl)propane, 2,2-bis(4-(meth)acryloyloxypentaethoxyphenyl)propane, 2,2-bis(4-(meth)acryloyloxydipropoxyphenyl)propane, 2-(4-(meth)acryloyloxydiethoxyphenyl)-2-(4-(meth)acryloyloxyethoxyphenyl)propane, 2-(4-(meth)acryloyloxydiethoxyphenyl)-2-(4-(meth)acryloyloxytriethoxyphenyl)propane, 2-(4-(meth)acryloyloxydipropoxyphenyl)-2-(4-(meth)acryloyloxytriethoxyphenyl)propane, 2,2-bis(4-(meth)acryloyloxypropoxyphenyl)propane, 2,2-bis(4-(meth)acryloyloxyisopropoxyphenyl)propane, and 1,4-bis(2-(meth)acryloyloxyethyl)pyromellitate. In view of excellent polymerizability and providing a zirconia molded body having superior mechanical strength, preferred are Bis-GMA, and 2,2-bis(4-(meth)acryloyloxypolyethoxyphenyl)propane. Preferred as 2,2-bis(4-(meth)acryloyloxypolyethoxyphenyl)propane is 2,2-bis(4-methacryloyloxypolyethoxyphenyl)propane (with an average of 2.6 moles of ethoxy group added; commonly known as D-2.6E).

Examples of the bifunctional aliphatic compound include (meth)acrylates such as glycerol di(meth)acrylate, ethylene glycol di(meth)acrylate, diethylene glycol di(meth)acrylate, triethylene glycol di(meth)acrylate, propylene glycol di(meth)acrylate, butylene glycol di(meth)acrylate, neopentyl glycol di(meth)acrylate, polyethylene glycol di(meth)acrylate, 1,3-butanediol di(meth)acrylate, 1,4-butanediol di(meth)acrylate, 1,6-hexanediol di(meth)acrylate, 2-ethyl-1,6-hexanediol di(meth)acrylate, 1,9-nonanediol di(meth)acrylate, 1,10-decanediol di(meth)acrylate, 1,2-bis(3-methacryloyloxy-2-hydroxypropoxy)ethane, and 2,2,4-trimethylhexamethylenebis(2-carbamoyloxyethyl)dimethacrylate (commonly known as UDMA). In view of excellent polymerizability and providing a zirconia molded body having superior mechanical strength, preferred are triethylene glycol dimethacrylate (commonly known as TEGDMA), and UDMA.

Examples of the tri- or higher-functional compound include (meth)acrylates such as trimethylolpropane tri(meth)acrylate, trimethylolethane tri(meth)acrylate, trimethylolmethane tri(meth)acrylate, pentaerythritol tri(meth)acrylate, pentaerythritol tetra(meth)acrylate, dipentaerythritol penta(meth)acrylate, N,N-(2,2,4-trimethylhexamethylene)bis[2-(aminocarboxy)propane-1,3-diol]tetra(meth)acrylate, and 1,7-diacryloyloxy-2,2,6,6-tetra(meth)acryloyloxymethyl-4-oxaheptane. In view of excellent polymerizability and providing a zirconia molded body having superior mechanical strength, preferred are N,N-(2,2,4-trimethylhexamethylene)bis[2-(aminocarboxy)propane-1,3-diol]tetramethacrylate, and 1,7-diacryloyloxy-2,2,6,6-tetraacryloyloxymethyl-4-oxaheptane.

In both of the foregoing methods, it is preferable that the composition be polymerized using a polymerization initiator, and that the composition additionally comprise a polymerization initiator. The polymerization initiator is not particularly limited. Particularly preferred are photopolymerization initiators. The photopolymerization initiator may be appropriately selected from photopolymerization initiators used in industry, preferably those used in dentistry. Specific examples of the photopolymerization initiator are no different from those mentioned above in the description of gel casting.

When the zirconia molded body is produced by stereolithography using the zirconia composition, the stereolithography method is not particularly limited to specific methods, and a known method may be appropriately selected for stereolithography. For example, it is possible to use a method that produces the desired zirconia molded body by successively forming layers in desired shapes through photopolymerization of a liquid composition with, for example, ultraviolet light or a laser, using a stereolithography device.

The zirconia molded body, as the cured product, may be subjected to a CIP process after a humidification process, in order to further improve the density of the zirconia molded body.

When press molding is performed, the zirconia particle-containing powder may be subjected to press molding after a humidification process, before pressing the powder. Any known methods can be used for the humidification process, including a method that sprays water with a sprayer or the like, and a method that uses a constant humidity chamber or a constant temperature and humidity chamber. The amount of moisture increase after the humidification process is preferably more than 2 mass%, more preferably more than 3 mass%, even more preferably more than 4 mass%, particularly preferably more than 5 mass%, and is preferably 15 mass% or less, more preferably 13 mass% or less, even more preferably 11 mass% or less relative to the mass of the unwetted powder (powder before humidification process) and molded body, though the amount of moisture increase depends on factors such as the average particle size of the zirconia particles and stabilizer particles contained. The amount of moisture increase by humidification process can be determined as a percentage by subtracting the mass of the unwetted powder and molded body from the mass of the wetted powder (powder after humidification process) and molded body, and dividing the difference by the mass of the unwetted powder and molded body.

The CIP process uses the same pressure as specified previously for press molding.

### (f) Additive Fabrication of Granules Containing Zirconia Particles and Yttria Particles

There is no specific restriction on the method for producing granules containing zirconia particles and yttria particles. For example, granules can be obtained by preparing a slurry and drying it with a spray dryer, and these granules may be used for powder additive fabrication.

The technique employed for powder additive fabrication is not particularly limited. Examples include the powder bed method, SLS method (selective laser sintering), SLM method (selective laser melting), electron beam method, arc discharge method, and binder jet method. For techniques where organic materials should be excluded in additive fabrication, it is preferable to also avoid using organic materials during the production of granules.

The next step is pre-sintering of the molded body to obtain a zirconia pre-sintered body of the present invention.

In view of ensuring a semi-sintered state using the specific zirconia composition above, the pre-sintering temperature (highest pre-sintering temperature) in the pre-sintering step is preferably 800°C or more, more preferably 850°C or more, even more preferably 900°C or more, particularly preferably 950°C or more.

In view of ensuring processability, the pre-sintering temperature is preferably 1,200°C or less, more preferably 1,150°C or less, even more preferably 1,100°C or less, particularly preferably 1,050°C or less.

That is, the preferred pre-sintering temperature is 800°C to 1,200°C in the method of production of a zirconia pre-sintered body of the present invention.

Concerning the hold time (retention time) at the highest pre-sintering temperature, the highest pre-sintering temperature is held for preferably 30 minutes to 6 hours to achieve a semi-sintered state.

The rate of temperature increase to the highest pre-sintering temperature, and the rate of temperature decrease from the highest pre-sintering temperature are preferably 300°C/min or less.

In certain preferred embodiments, the rate of temperature increase to the highest pre-sintering temperature in pre-sintering of a zirconia molded body of the present invention is not particularly limited. However, the rate of temperature increase to the highest pre-sintering temperature is preferably 0.1°C/min or more, more preferably 0.2°C/min or more, even more preferably 0.5°C/min or more, and is preferably 50°C/min or less, more preferably 30°C/min or less, even more preferably 20°C/min or less.

The productivity improves when the rate of temperature increase is at or above these lower limits. When the rate of temperature increase is at or below the foregoing upper limits, it is possible to reduce the volume difference between the inner and outer portions of the zirconia molded body and zirconia pre-sintered body. Additionally, when the zirconia molded body contains organic materials, the rapid decomposition of these organic materials can be reduced to inhibit cracks or fractures.

### [Zirconia Sintered Body]

The following describes the zirconia sintered body.

The zirconia sintered body is obtained by firing the zirconia pre-sintered body obtained in the manner described above.

The stabilizer content (the content of yttria and stabilizers other than yttria) specified for the zirconia pre-sintered body is also applicable to a zirconia sintered body of the present invention.

A zirconia sintered body of the present invention may comprise a fluorescent agent. The fluorescent agent may be the same fluorescent agent used in the zirconia pre-sintered body.

In this specification, concerning the fluorescent agent, the phrase "relative to 100 mass% of zirconia contained in zirconia pre-sintered body" can be read as "relative to 100 mass% of zirconia contained in zirconia sintered body."

A zirconia sintered body of the present invention may comprise a colorant. The colorant may be the same colorant used in the zirconia pre-sintered body.

The colorant content in the zirconia sintered body is not particularly limited, and may be appropriately adjusted according to factors such as the type of colorant, and intended use of the zirconia sintered body. However, in view of considerations such as suitability in dental prosthesis applications, the colorant content is preferably 0.001 mass% or more, more preferably 0.005 mass% or more, even more preferably 0.01 mass% or more in terms of the oxide equivalent of the metal elements contained in the colorant, relative to 100 mass% of zirconia contained in the zirconia sintered body. The colorant content is preferably 5 mass% or less, more preferably 1 mass% or less, even more preferably 0.5 mass% or less in terms of the oxide equivalent of the metal elements contained in the colorant. The colorant content may be 0.1 mass% or less, or 0.05 mass% or less.

A zirconia sintered body of the present invention may comprise a translucency adjuster, in order to adjust the translucency of the zirconia sintered body. The translucency adjuster may be the same translucency adjuster used in the zirconia pre-sintered body.

The content of the translucency adjuster in the zirconia sintered body is not particularly limited, and may be appropriately adjusted according to factors such as the type of translucency adjuster, and intended use of the zirconia sintered body. However, in view of considerations such as suitability in dental prosthesis applications, the content of the translucency adjuster is preferably 0.1 mass% or less relative to 100 mass% of zirconia contained in the zirconia sintered body.

The relative density of the zirconia sintered body is preferably 99.0% or more, more preferably 99.2% or more, even more preferably 99.5% or more.

The relative density can be calculated as a ratio of the actual density, measured by the Archimedes method, with respect to the theoretical density.

A greater density in the zirconia sintered body leads to fewer internal voids and less scattering of light, and thereby improves translucency. From this perspective, the relative density is preferably 5.80 g/cm³ or more, more preferably 5.82 g/cm³ or more, even more preferably 5.87 g/cm³ or more. Particularly preferably, the zirconia sintered body is essentially void free.

The zirconia content and the stabilizer content specified for the composition and/or pre-sintered body before preparation into the sintered body are also applicable to the zirconia sintered body.

It is preferable for the zirconia sintered body to exhibit higher strength. For example, the strength is preferably 800 MPa or more, more preferably 820 MPa or more, even more preferably 840 MPa or more in terms of a biaxial flexural strength. The biaxial flexural strength can be measured in compliance with ISO 6872: 2015.

### [Production Method of Zirconia Sintered Body]

A zirconia sintered body of the present invention can be produced by, for example, a method that fires the zirconia pre-sintered body.

Preferably, the method comprises the step of firing the zirconia pre-sintered body at 1,350°C or more and 1,700°C or less under ordinary pressure. By employing such a method, a zirconia sintered body of the present invention can be easily produced that can achieve a relative density of 99.0% or more and exhibit excellent translucency even after fast firing.

The production method of the present invention also enables easy production of a zirconia sintered body of the present invention that can exhibit excellent translucency even when sintered for a short period with a hold time of 10 minutes or less (for example, 2 minutes) at the highest sintering temperature.

When the sintered body is produced by firing a zirconia pre-sintered body of the present invention, it is preferable to employ highest sintering temperature conditions that maximize the translucency of the zirconia sintered body.

In view of considerations such as allowing easy production of the desired zirconia sintered body under ordinary pressure, the highest sintering temperature is preferably more than 1,200°C, more preferably 1,250°C or more, even more preferably 1,300°C or more. The highest sintering temperature is preferably 1,700°C or less, more preferably 1,650°C or less, even more preferably 1,600°C or less.

When the highest sintering temperature is at or above the foregoing lower limits and at or below the upper limits specified above, the sintering process can sufficiently proceed, easily yielding a dense sintered body. When the highest sintering temperature is at or below the foregoing upper limits, it is possible to inhibit deactivation of the fluorescent agent.

When producing the sintered body, the hold time at the highest sintering temperature is not particularly limited. However, in view of considerations such as enabling efficient and stable production of the desired zirconia sintered body with good productivity, the hold time at the highest sintering temperature is preferably 10 minutes or less, more preferably 5 minutes or less, even more preferably 3 minutes or less, particularly preferably 2 minutes or less.

The hold time is preferably 30 seconds or more, more preferably 45 seconds or more, even more preferably 1 minute or more.

Preferably, the rate of temperature decrease in the firing process is set so as to reduce the time required for the firing process. For example, the rate of temperature increase may be set so that the highest sintering temperature is reached in the shortest possible time, depending on the capabilities of the furnace. Preferably, the rate of temperature decrease from the highest sintering temperature is set to a rate that does not cause defects, such as cracks, in the sintered body. For example, the sintered body may be allowed to cool at room temperature after the heating is finished.

In the present invention, a firing furnace can be used for pre-sintering and firing. The type of furnace is not particularly limited, and may be, for example, a furnace used in industry, such as an electric furnace or debinding furnace.

It is also possible to use a commercially available dental firing furnace (for example, Sintra CS manufactured by Shenpaz under this trade name).

A zirconia sintered body of the present invention can be produced even without a HIP process. However, the translucency and mechanical strength can further improve when a HIP process, as an optional process, follows the sintering performed under ordinary pressure.

In the following, the term "primary sintered body" is used to refer to a sintered body obtained through sintering at the highest sintering temperature (sintered body before HIP processing), whereas "HIP sintered body" is used to refer to a sintered body after HIP processing.

Known hot isostatic pressing (HIP) devices can be used for HIP processing.

The HIP pressure in the HIP process of the primary sintered body is not particularly limited. However, for advantages such as obtaining a dense sintered body with high strength, the HIP pressure is preferably 100 MPa or more, more preferably 125 MPa or more, even more preferably 130 MPa or more. The upper limit of HIP pressure is not particularly limited, and may be, for example, 400 MPa or less, 300 MPa or less, or 200 MPa or less.

The rate of temperature increase in the HIP process of the primary sintered body is not particularly limited, and is preferably 0.1°C/min or more, more preferably 0.2°C/min or more, even more preferably 0.5°C/min or more. The rate of temperature increase is preferably 50°C/min or less, more preferably 30°C/min or less, even more preferably 20°C/min or less. Productivity improves when the rate of temperature increase is at or above the foregoing lower limits.

The duration of HIP in the HIP process of the primary sintered body (the duration in which the highest pressure and temperature are maintained) is not particularly limited. However, for advantages such as obtaining a dense zirconia sintered body with high strength, the duration of the HIP process is preferably 5 minutes or more, more preferably 10 minutes or more, even more preferably 30 minutes or more. The duration of the HIP process is preferably 10 hours or less, more preferably 6 hours or less, even more preferably 3 hours or less.

In the method for producing a zirconia sintered body of the present invention, the pressure medium in the HIP process of the primary sintered body is not particularly limited. However, in view of a low impact on zirconia, the pressure medium may be at least one selected from the group consisting of oxygen, oxygen with 3% hydrogen, air, and various inert gases (for example, nitrogen, argon).

When conducting HIP processing of the primary sintered body in an oxygen-mixed gas atmosphere, the oxygen concentration may be, for example, more than 0% and 20% or less, though it is not particularly limited.

When using an oxygen-mixed gas, at least one inert gas (for example, such as nitrogen or argon) may be selected as a gas other than oxygen.

The HIP process may result in blackening due to oxygen deficiency when the HIP process is carried out in a reducing atmosphere such as by using an inert gas. In order to prevent such blackening, it is preferable to include a heat treatment step (or tempering as it is also called hereinbelow), performed at 1,650°C or less in either the atmosphere or an excess oxygen atmosphere, after the HIP process. In view of the efficiency of heat treatment, the heat treatment step is more preferably carried out in an excess oxygen atmosphere.

Here, excess oxygen atmosphere means an atmosphere where the oxygen concentration exceeds that of the atmosphere.

The excess oxygen atmosphere is not particularly limited, as long as the oxygen concentration is more than 21% and 100% or less, and can be appropriately selected within this range. For example, the oxygen concentration may be 100%. The HIP process may result in blackening due to oxygen deficiency when the HIP process is carried out in a reducing atmosphere such as by using an inert gas. In order to prevent such blackening, it is preferable to include a heat treatment step (or tempering as it is also called hereinbelow), performed at 1,650°C or less in either the atmosphere or an excess oxygen atmosphere, after the HIP process. In view of the efficiency of heat treatment, the heat treatment step is more preferably carried out in an excess oxygen atmosphere. Here, excess oxygen atmosphere means an atmosphere where the oxygen concentration exceeds that of the atmosphere. The excess oxygen atmosphere is not particularly limited, as long as the oxygen concentration is more than 21% and 100% or less, and can be appropriately selected within this range. For example, the oxygen concentration may be 100%.

A zirconia sintered body of the present invention may be a primary sintered body, a HIP sintered body, or a tempered sintered body with no particular restriction, provided that the present invention can exhibit its effects.

The heat treatment temperature in the atmosphere or an excess oxygen atmosphere may be appropriately modified according the aesthetics of the zirconia sintered body (for example, the shade of dental prostheses).

In a certain preferred embodiment, in view of the aesthetics of the zirconia sintered body, the heat treatment temperature in the atmosphere or an excess oxygen atmosphere is preferably 1,650°C or less, more preferably 1,600°C or less, even more preferably 1,550°C or less.

In another preferred embodiment, in view of the aesthetics of the zirconia sintered body, the heat treatment temperature in the atmosphere or an excess oxygen atmosphere is preferably 1,400°C or less, more preferably 1,300°C or less, even more preferably 1,200°C or less.

In either embodiment, the heat treatment temperature is preferably 500°C or more, more preferably 600°C or more, even more preferably 700°C or more.

A common dental zirconia furnace can be used for the tempering process. The dental zirconia furnace may be a commercially available product. Examples of commercially available products include Noritake KATANA^{®} F-1, F-1N, and F-2 (SK Medical Electronics Co., Ltd.).

The zirconia sintered body produced by firing a zirconia pre-sintered body of the present invention can be suitably used as a dental product.

Examples of such dental products include copings, frameworks, crowns, crown bridges, abutments, implants, implant screws, implant fixtures, implant bridges, implant burs, brackets, denture bases, inlays, onlays, orthodontic wires, and laminate veneers.

Using a zirconia pre-sintered body of the present invention for components such as implant screws and implant fixtures can reduce gingival discoloration associated with the use of metallic materials, thereby enhancing aesthetics.

Additionally, a suitable producing method can be chosen depending on the intended application. For example, a dental product can be obtained by firing a zirconia pre-sintered body of the present invention after it has been machined. The use of a CAD/CAM system is preferred for the machining process.

The CAD/CAM system is not particularly limited, and known devices may be used. Examples of such devices include known CAD/CAM systems such as the KATANA^{®} CAD/CAM system manufactured by Kuraray Noritake Dental Inc.

The present invention encompasses embodiments combining all or part of the foregoing features in various ways within the technical idea of the present invention, provided that the present invention can exhibit its effects.

### EXAMPLES

The present invention is described below in greater detail through Examples. It is to be noted, however, that the present invention is in no way limited by the following Examples, and various modifications may be made by a person with ordinary skill in the art within the technical idea of the present invention.

### [Example 1]

A commercially available zirconia powder (Y₂O₃: 0 mol%) and a commercially available yttria powder were charged into water. The mixture, along with a pulverization medium made of zirconia (diameter: 2 mm), was placed in the vessel of a ball mill, and pulverized for 96 hours with the ball mill to obtain a slurry (average particle diameter: 0.2 µm or less).

Subsequently, an organic binder was added into the slurry. After mixing and stirring, the slurry was dried and granulated with a spray dryer to obtain a powder.

The powder was poured into a columnar mold, and uniaxially pressed at 200 MPa pressure to obtain a molded body.

The molded body was placed in an electric furnace, heated from room temperature at 10°C/min, and retained at 500°C for 2 hours to debind the organic components. The resulting material was then heated at 10°C/min, held at 955°C for 2 hours, and allowed to cool at -10°C/min to obtain a zirconia pre-sintered body.

### [Example 2 and Comparative Examples 1 and 2]

The zirconia pre-sintered body was produced following the same method described in Example 1, except that the conditions indicated in Table 1 were applied. Example 2 and Comparative Examples 1 and 2 used the same raw material for yttria as that used in Example 1.

**[Table 1]**

| | Yttria content (mol%) | Raw materials used | Pulverization device | Pulverization time (h) | Maximum amount of change in the rate of density change | ΔL*(W-B) (thickness: 1 mm) | Relative density (%) |
|---|---|---|---|---|---|---|---|
| Ex. 1 | 5.5 | Monoclinic zirconia + yttria | Ball mill | 96 | 0.011 | 16 | 99.3 |
| Ex. 2 | 5.5 | Monoclinic zirconia + yttria | Ball mill | 168 | 0.016 | 16 | 99.5 |
| Com. Ex. 1 | 3.0 | Commercially available product Zpex | - | 0 | 0.009 | 7 | 98.3 |
| Com. Ex. 2 | 5.5 | Monoclinic zirconia + yttria | Ball mill | 20 | 0.001 | 10 | 98.5 |

In the table, the yttria content refers to the mole percentage of yttria relative to the total number of moles of zirconia and yttria (mol%).

In the table, the symbol "-" means that the pulverization process was not performed.

The yttria content represents the value in the slurry, molded body, pre-sintered body, and sintered body.

In the table, the commercially available product in Comparative Example 1 is the Zpex manufactured by Tosoh Corporation under this trade name.

### <Method for Measuring Density and Calculating the Rate of Density Change in the Production of Zirconia Sintered Body>

The zirconia pre-sintered bodies of Examples 1 and 2 and Comparative Examples 1 and 2 were pulverized under the conditions given in Table 1, and molded into disc-shaped molded bodies with a diameter of approximately 18 mm and a thickness of about 1.2 mm. The molded bodies were held for 2 hours at a highest pre-sintering temperature of 1,000°C, and allowed to cool at -0.4°C/min to obtain zirconia pre-sintered bodies.

From 1,150°C to 1,400°C, six target temperatures were set at 50°C intervals. The zirconia pre-sintered body of each Example and Comparative Example was then heated from room temperature to each target temperature according to the theoretical density, using a dental furnace (Sintra CS, manufactured by Shenpaz under this trade name). The zirconia pre-sintered body was retained at the target temperature for 10 minutes, and cooled to 25°C to obtain six samples (zirconia sintered bodies) corresponding to the different target temperatures (highest sintering temperatures). During cooling, the rate was set at -200°C/min down to 950°C, after which the furnace was opened to allow for natural cooling. All samples were cooled under the same conditions.

The dimensions of each sample were measured with accuracy, using an electronic balance to find the mass of the zirconia sintered body, and a micrometer to find the thickness and diameter. Using the measured mass from the precision balance, the density was determined by dividing the mass of the pre-sintered body by its volume (arithmetic mean value for n = 3).

The amount of change between the rate of density change Δρₙ/ΔT in the nth interval and the rate of density change Δρₙ₊₁/ΔT in the (n+1)th interval was then calculated in the temperature range of 1,200°C or more and 1,350°C or less, where Δρₙ represents a change in density of the pre-sintered body between the upper-limit temperature and the lower-limit temperature of the nth interval (n being an integer of 1 or greater), and ΔT represents a change in temperature as defined for the six samples in the temperature range of target temperatures from 1,150°C to 1,400°C divided into 50°C intervals. The results are presented in Table 2.

**[Table 2]**

| | | Ex. 1 | | Ex. 2 | | Com. Ex. 1 | | Com. Ex. 2 | |
|---|---|---|---|---|---|---|---|---|---|
| Temperature (°C) | nth interval | Density | Amount of change in the rate of density change | Density | Amount of change in the rate of density change | Density | Amount of change in the rate of density change | Density | Amount of change in the rate of density change |
| 1150 | First interval | 3.38 | | 3.41 | | 3.89 | | 3.37 | |
| 1200 | Second interval | 3.73 | 0.006 | 3.78 | 0.004 | 4.86 | -0.001 | 3.55 | 0.004 |
| 1250 | Third interval | 4.38 | 0.011 | 4.33 | 0.016 | 5.80 | -0.016 | 3.95 | 0.009 |
| 1300 | Fourth interval | 5.56 | -0.017 | 5.69 | -0.022 | 5.94 | -0.003 | 4.80 | 0.003 |
| 1350 | Fifth interval | 5.91 | -0.006 | 5.97 | -0.005 | 5.96 | 0.001 | 5.81 | -0.018 |
| 1400 | | 5.97 | | 6.00 | | 6.02 | | 5.94 | |

| | | | | | | | | | |
|---|---|---|---|---|---|---|---|---|---|
| In the table, the unit of density is g/cm³. | | | | | | | | | |

### <Evaluation of Translucency of Zirconia Sintered Body (Measurement of ΔL*(W-B))>

The zirconia pre-sintered body of each Example and Comparative Example was subjected to short firing involving a 2-minute retention time at 1,580°C with a rate of temperature increase of 350°C/min, using a dental furnace (Sintar CS manufactured by Shenpaz under this trade name).

The resulting zirconia sintered body was polished into a flat plate sample with a thickness of 1.0 mm and used for translucency measurement. The translucency measurement was carried out using a spectrophotometer (Crystaleye manufactured by Olympus Corporation under this trade name) in 7-band measurement mode with an LED light source.

Specifically, the translucency of the zirconia sintered body, prepared as a flat plate sample, was measured by finding the lightness (LW*) and lightness (LB*) from chromaticity measurement against a white background and a black background, respectively, using the same sample. The measurements were carried out using the same measurement instrument in the same measurement mode with the same light source. The difference between these values (ΔL* = (LW*) - (LB*)) was then determined as the translucency (ΔL*(W-B)) (arithmetic mean value for n = 3). The L* value is an L* value in the chromaticity (color space) of the L*a*b* color system (JIS Z 8781-4:2013).

As a translucency ΔL*(W-B) of the sintered body, a value of 13 or greater was considered acceptable. The results are presented in Table 1.

### <Method for Calculating Relative Density of Zirconia Sintered Body>

Using a dental furnace (Sintar CS manufactured by Shenpaz under this trade name), the zirconia pre-sintered body of each Example and Comparative Example was subjected to short firing with a 2-minute retention time at 1,580°C, with a rate of temperature increase of 350°C/min.

The resulting zirconia sintered body was then polished into a flat plate sample with a thickness of 1.0 mm and used for density measurement. The actual density was then measured using the Archimedes method.

The relative density was calculated as a ratio of the actual density, measured by the Archimedes method, with respect to the theoretical density.

The results are presented in Table 1.

As demonstrated above, the zirconia pre-sintered body of the present invention achieved a relative density of 99.0% or more in fast firing with a high rate of temperature increase of 350°C/min, and yielded a zirconia sintered body exhibiting excellent translucency.

Comparative Examples failed to produce a zirconia sintered body achieving theoretical density and excellent translucency.

### INDUSTRIAL APPLICABILITY

A zirconia pre-sintered body of the present invention is useful for the production of dental products used in dental treatments at dental clinics.

## Claims

1. A zirconia pre-sintered body comprising zirconia and yttria,
wherein the zirconia pre-sintered body, with six target temperatures set from 1,150°C to 1,400°C at 50°C intervals and six samples prepared by heating the zirconia pre-sintered body from a temperature range of 400°C or less to each different target temperature at a rate of temperature increase of 350°C/min, followed by 10-minute retention at the target temperature and subsequent cooling to 25°C, has a transition point where the maximum value of the amount of change ((Δρₙ₊₁/ΔT) - (Δρₙ/ΔT)) between the rate of density change Δρₙ/ΔT in the nth interval and the rate of density change Δρₙ₊₁/ΔT in the (n+1)th interval becomes 0.010 or more in the temperature range of 1,200°C or more and 1,350°C or less, where Δρₙ represents a change in density of the pre-sintered body between the upper-limit temperature and the lower-limit temperature of the nth interval (n being an integer of 1 or greater), and ΔT represents a change in temperature as defined for the six samples in the temperature range of target temperatures from 1,150°C to 1,400°C divided into 50°C intervals.

2. The zirconia pre-sintered body according to claim 1, wherein the yttria content is 3.5 mol% or more and 7.5 mol% or less relative to the total moles of the zirconia and the yttria.

3. The zirconia pre-sintered body according to claim 1 or 2, which has a density of 4.5 g/cm³ or less at the temperature (°C) of the transition point.
